Europäisches Patentamt

European Patent Office

Office européen des brevets

(19)

(11) Veröffentlichungsnummer: **0 283 765**
**B1**

# (12) EUROPÄISCHE PATENTSCHRIFT

(45) Veröffentlichungstag der Patentschrift:
08.08.90

(21) Anmeldenummer: 88103023.3

(22) Anmeldetag: 29.02.88

(51) Int. Cl.⁵: **G01N 23/04**, G01V 5/00,
H05G 1/54

(54) **Röntgenscanner.**

(30) Priorität: 11.03.87 DE 8703674 U

(43) Veröffentlichungstag der Anmeldung:
28.09.88 Patentblatt 88/39

(45) Bekanntmachung des Hinweises auf die Patenterteilung:
08.08.90 Patentblatt 90/32

(84) Benannte Vertragsstaaten:
DE FR GB NL

(56) Entgegenhaltungen:
DE-C- 933 051
DE-C- 967 931
GB-A- 2 110 037
US-A- 4 217 064
US-A- 4 326 131

PATENT ABSTRACTS OF JAPAN, Band 10,
Nr. 289 (E-442)[2345], 20. Oktober 1986; &
JP-A-61 107 698 (HITACHI MEDICAL CORP.) 26-05-1986

(73) Patentinhaber: Heimann GmbH, Weher
Köppel 6 Postfach 30 07, D-6200 Wiesbaden 1(DE)

(72) Erfinder: Dönges, Gerhard, Dipl.-Ing., Am Markt 1,
D-6209 Heidenrod-Kemel(DE)
Erfinder: Dietrich, Rolf, Dipl.-Ing., Hölderlinstrasse 4,
D-6238 Hofheim(DE)
Erfinder: Thoma, Helmut, L.-Bamberger Strasse 16,
D-6500 Mainz(DE)
Erfinder: Von Unger, Eckart-Alfred, Dipl.-Ing.,
Kuglmüllerstrasse 22, D-8000 München 19(DE)

(74) Vertreter: Fuchs, Franz-Josef, Dr.-Ing. et al,
Postfach 22 13 17, D-8000 München 22(DE)

## Beschreibung

Die Erfindung betrifft einen Röntgenscanner mit einem Röntgenstrahler mit Kollimator für ein fächerförmiges Röntgenstrahlenbündel auf einer Seite und einem Strahlenempfänger auf der anderen Seite einer Transportvorrichtung für zu untersuchendes Gut sowie mit einer Elektronik zur Erfassung und Verarbeitung der Detektorsignale und einem nachgeschalteten Sichtgerät.

Ein Röntgenscanner dieser Art dient beispielsweise zur Gepäckuntersuchung. Der Strahlenempfänger kann dabei von einer Reihe von Einzeldetektoren gebildet sein, die parallel der jeweils gemessenen Strahlungsintensität nach dem Prüfgut entsprechende Meßwerte liefern, welche in einen Bildspeicher eingeschrieben werden. Bei der Darstellung des Speicherinhaltes auf dem Sichtgerät entsteht dabei ein durchlaufendes Bild.

Bei einem Röntgenscanner der genannten Art ist die Qualität der vom Strahlenempfänger erzeugten Signale entscheidend von der genauen Justierung des fächerförmigen Röntgenstrahlenbündels abhängig. Maßgebend ist eine exakte geometrische Zuordnung der Komponenten Fokus - Kollimator - Strahlenempfänger. Um die Strahlendosis und die Streustrahlung auf ein Mindestmaß zu beschränken, sollte mit möglichst dünnen Strahlenfächern und damit möglichst kleinen Spaltbreiten des Kollimators von in der Regel einigen Zehntel Millimetern gearbeitet werden. Entsprechend den geometrischen Verhältnissen (Abstand Fokus - Kollimator und Kollimator - Strahlenempfänger) ergibt sich dabei eine etwa drei Millimeter breite Bestrahlung des Strahlenempfängers, so daß dieser gerade vollständig von Röntgenstrahlung getroffen wird. Besonders beim Einsatz von als geknickte Detektorzeilen ausgebildeten Strahlenempfängern muß eine sehr genaue Justierung erfolgen.

Durch die Bewegung der Transportvorrichtung während des Betriebes und durch das Auflegen von Gepäckstücken und besonders beim Einsatz in Fahrzeugen als fahrbarer Röntgenscanner ist der Röntgenscanner in erheblichem Maße Stößen und Schwingungen ausgesetzt. Aus diesem Grund ist die Gefahr der Dejustierung beim Transport oder durch beim Betrieb auftretende Erschütterungen groß.

Der Erfindung liegt demgemäß die Aufgabe zugrunde, einen Röntgenscanner der eingangs genannten Art so auszubilden, daß die Gefahr der Dejustierung durch die obengenannten Einflüsse reduziert ist.

Diese Aufgabe ist erfindungsgemäß dadurch gelöst, daß der Röntgenstrahler, der Kollimator und der Strahlenempfänger an einem gemeinsamen, biege- und verdrehsteifen Rahmen befestigt sind, welcher im Gestell des Röntgenscanners elastisch gelagert ist. Bei dieser Ausbildung kann die Transportvorrichtung, die vorzugsweise ein Transportband ist, mechanisch von dem Rahmen getrennt werden, so daß sich während des Betriebes Stöße und Schwingungen auch wegen der elastischen Lagerung des Rahmens kaum auf diesen auswirken. Auch bei Stößen auf das Gerät von außen ist der Rahmen aufgrund seiner elastischen Lagerung geschützt.

Eine zweckmäßige Weiterbildung der Erfindung besteht darin, daß der Kollimator und der Röntgenstrahler individuell am Rahmen befestigt sind. Bei dieser Weiterbildung bleibt im Servicefall beim Austausch des Röntgenstrahlers die genaue Zuordnung des Kollimators zum Strahlenempfänger erhalten. Es muß lediglich der Röntgenstrahler neu justiert werden, was relativ einfach ist.

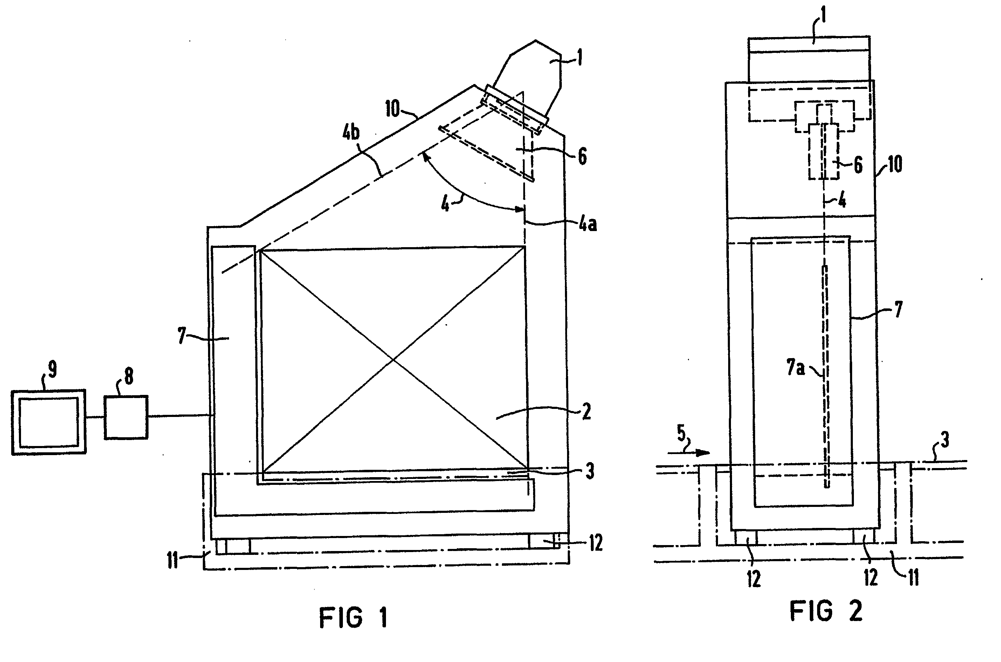

Die Erfindung ist nachfolgend anhand eines in den Fig. 1 und 2 in zwei Ansichten dargestellten Ausführungsbeispieles näher erläutert.

In der Zeichnung ist ein Röntgenstrahler 1 dargestellt, der Prüfgut 2 (in Fig. 2 nicht dargestellt) auf einem Transportband 3 mit einem fächerförmigen Röntgenstrahlenbündel 4 durchstrahlt, dessen Fächerebene quer zur Transportrichtung liegt, welche in der Fig. 1 senkrecht zur Zeichenebene und in der Fig. 2 in Richtung des Pfeiles 5 verläuft. Zur Einblendung des fächerförmigen Röntgenstrahlenbündels 4 ist ein aus Bleiplatten bestehender Kollimator 6 vorgesehen. Die Randstrahlen des Röntgenstrahlenbündels 4 sind mit 4a und 4b bezeichnet. Die aus dem Prüfgut 2 austretende Strahlung wird von einem rechtwinklig geknickten Strahlenempfänger 7 aus einzelnen Detektoren in einem Gehäuse erfaßt, deren Ausgangssignale über eine Elektronik 8 einem Sichtgerät 9 zugeführt werden. Der Erfassungsbereich für die Strahlung im Strahlenempfänger ist in Fig. 2 mit 7a bezeichnet. Die Elektronik 8 bildet aus den Ausgangssignalen des Strahlenempfängers 7 ein Übersichtsbild eines Bereiches des Prüfgutes 2, das auf dem Sichtgerät 9 dargestellt wird.

Der Röntgenstrahler 1, der Strahlenempfänger 7 und der Kollimator 6 sind an einem gemeinsamen, biege- und verdrehsteifen Rahmen 10 befestigt, welcher im Gestell 11 des Röntgenscanners mit Hilfe von Metallgummi-Federelementen 12 elastisch gelagert ist, so daß mechanische Stöße und Schwingungen gedämpft werden.

Der Kollimator 6 und der Röntgenstrahler 1 sind jeweils individuell am Rahmen 10 befestigt. Der Röntgenstrahler 1 kann demgemäß ausgetauscht werden, wobei die Ausrichtung des Kollimators 6 auf den Strahlenempfänger 7 erhalten bleibt.

Der Strahlenempfänger 7 kann in bekannter Weise aus einer geknickten Reihe von Einzeldetektoren bestehen, wobei jeder Einzeldetektor von einem Szintillationskristall und einer nachgeschalteten Photodiode gebildet ist.

## Patentansprüche

1. Röntgenscanner mit einem Röntgenstrahler (1) mit einem Kollimator (6) für ein fächerförmiges Röntgenstrahlenbündel (4) auf einer Seite und einem Strahlenempfänger (7) auf der anderen Seite einer Transportvorrichtung (3) für Prüfgut (2) sowie mit einer Elektronik (8) zur Erfassung und Verarbeitung der Detektorsignale und einem nachgeschalteten Sichtgerät (9), dadurch gekennzeichnet, daß der Röntgenstrahler (1), der Kollimator (6) und der Strahlenempfänger (7) an einem gemeinsamen, biege- und verdrehsteifen Rahmen (10) befestigt sind, welcher im Gestell (11) des Röntgenscanners elastisch gelagert ist.

2. Röntgenscanner nach Anspruch 1, dadurch gekennzeichnet, daß der Kollimator (6) und der Röntgenstrahler (1) individuell am Rahmen (10) befestigt sind.

## Claims

1. An x-ray scanner having an x-ray source (1) with a collimator (6) for a fan-shaped x-ray beam (4) on one side of a transporting device (3) for an article to be examined (2) and on the other side a radiation receiver (7) as well as having an electronic system (8) for detecting and processing the detector signals and a downstream visual display (9), characterised in that the x-ray source (1), the collimator (6) and the radiation receiver (7) are secured to a common, bending-resistant and twistingresistant frame (10), which is flexibly mounted in the support (11) of the x-ray scanner.

2. An x-ray scanner according to claim 1, characterised in that the collimator (6) and the x-ray source (1) are secured individually on the frame (10).

## Revendications

1. Scanner X comportant un émetteur (1) de rayons X pourvu, d'un côté d'un dispositif de transport (3) de l'objet à contrôler (2), d'un collimateur (6) servant à produire un faisceau de rayons X en forme d'éventail (4), et de l'autre côté du dispositif de transport, un récepteur de rayonnement (7), et comportant un système électronique (8) servant à détecter et à traiter les signaux provenant de détecteurs et un appareil de visualisation (9) branché en aval, caractérisé par le fait que l'émetteur (1) de rayons X, le collimateur (6) et le récepteur de rayonnement (7) sont fixés sur un cadre commun (10) résistant à la flexion et à la torsion, qui est supporté élastiquement dans le châssis (11) du scanner X.

2. Scanner X suivant la revendication 1, caractérisé par le fait que le collimateur (6) et l'émetteur (1) de rayons X sont fixés individuellement sur le cadre (10).

FIG 1

FIG 2

EP 0 283 765 B1